# EUROPEAN PATENT APPLICATION

(11) **EP 2 468 309 A1**
(43) Date of publication of application: **27.06.2012**
(21) Application number: 11157083.4
(22) Date of filing: 04.03.2011
(51) Int. Cl.: A61L 15/46, A61L 15/28

(54) **Absorbent article having releasable odor control**

(30) Priority: 21.12.2010 EP 10015886; 21.12.2010 EP 10015887
(71) Applicant: The Procter & Gamble Company, Cincinnati, OH 45202 (US)
(72) Inventor: Caputi, Mariangela, 65126 Pescara (IT); Pourcel, Magali, 65125 Pescara (IT); Tordone, Alessandra, 65125 Pescara (IT)
(74) Representative: Briatore, Andrea

(57) **Abstract**

An absorbent article comprising a cyclodextrin complex comprising cyclodextrin and at least two components complexed with said cyclodextrin, wherein said at least two components are:
a) a menthol family compounds
b) an ionone.

The absorbent articles of the invention are effective in preventing malodors associated with their use and do not exhibit perceptible odor before use.

## Description

### FIELD OF THE INVENTION

The present invention relates to an absorbent article comprising a cyclodextrin complex comprising cyclodextrin and a menthol family compound and an ionone both complexed with the cyclodextrin.

### BACKGROUND OF THE INVENTION

Absorbent articles for personal hygiene are known in the art. Typical examples include sanitary napkins, pantiliners, tampons, inter labial articles, adult incontinence articles, and baby diapers. Such articles are commonly used to absorb and retain bodily fluids and other exudates excreted by the human body, such as urine and menses. Typically, such exudates are perceived as malodorous and offensive. Therefore, methods and materials for controlling and reducing malodors in absorbent articles have been developed. Fragrance materials have been widely used for this purpose in absorbent articles, as well as ingredients such as silica or zeolites which are able to entrap some of the malodour generating molecules. The use of fragrance materials, however, tends to provide an overwhelming perfume scent to the product before use that may not be acceptable to some consumers. There thus still remains a desire to provide an improved malodor control composition for incorporation into an absorbent article product.

There remains a need to develop a malodor control technology that does not impart a perceptible odor to the absorbent article before use and that efficiently provides malodor control benefits throughout the period of time that the absorbent article is typically worn by a consumer.

### SUMMARY OF THE INVENTION

The present invention relates to absorbent articles comprising a cyclodextrin complex comprising cyclodextrin and at least two components complexed with said cyclodextrin, wherein said at least two components are:
a) a menthol family compounds selected from menthol, menthyl acetate, menthyl lactate, menthyl propionate, menthyl butyrrate, menthone, mint terpenes, laevo-carvone, Cis-3-Hexenol & Cis-3-Hexenyl acetate and mixture thereof,
b) an ionone selected from:
   1-(2,6,6-trimethyl-1-cyclohexenyl)pent-1-en-3-one;
   4-(2,6,6-trimethyl-1-cyclohexen-1-yl)-3-Buten-2-one;
   4-(2,6,6-trimethyl-2-cyclohexen-1-yl)-3-buten-2-one,
   5-(2,6,6-Trimethyl-2-cyclohexen-1-yl)4-penten-3-one,
   (E)-4-(2,2-dimethyl-6-methylidenecyclohexyl)but-3-en-2-one
   and mixture thereof.

The absorbent article of the present invention exhibits no, or very little, scent prior to use. Upon use, the bodily fluid contacts the cyclodextrin complex and provides an effective release of the components complexed within the cyclodextrin in order to reduce the malodor associated with the bodily fluid. The present invention can provide sustained odor control for the period of time the absorbent article is typically worn by a consumer, which is typically about 4 hours during the daytime and typically about 8 hours overnight.

The present invention further relates to a method of reducing the malodor associated with bodily fluids, such as urine, menses, and/or feces, comprising the step of contacting the bodily fluid with an absorbent article of the present invention.

### BRIEF DESCRIPTION OF THE DRAWINGS

FIG. 1 is a top view of an absorbent article of the present invention.
FIG. 2 is a cross-sectional view of the absorbent article of FIG. 1.

### DETAILED DESCRIPTION OF THE INVENTION

"Absorbent article" refers to devices that absorb and contain body exudates, such as urine, menses, and feces. The term "disposable" is used herein to describe absorbent articles which are not intended to be laundered or otherwise restored or reused as an absorbent article after a single use. Examples of absorbent articles include diapers, toddler training pants, adult incontinence garments, and feminine hygiene garments such as sanitary napkins, pantiliners, tampons, interlabial devices, hemorrhoid pads, and the like.

Absorbent articles and components thereof, including the topsheet, backsheet, absorbent core, and any individual layers of these components, can have a body-facing surface and a garment-facing surface. As used herein, "body-facing surface" means that surface of the article or component which is intended to be worn toward or adjacent to the body of the wearer, while the "garment-facing surface" is on the opposite side and is intended to be worn toward or placed adjacent to the wearer's undergarments when the disposable absorbent article is worn.

### ABSORBENT ARTICLE

Most absorbent articles of the present invention (except those for internal use such as tampons) typically comprise a topsheet, a backsheet, and an absorbent core disposed between the topsheet and backsheet.

The topsheet of the absorbent article is preferably compliant, soft feeling, and nonirritating to the wearers skin and hair. Further, the topsheet is liquid pervious, permitting liquids (e.g., menses and/or urine) to readily penetrate through its thickness. A suitable topsheet may be manufactured from a wide range of materials such as woven and nonwoven materials (e.g., a nonwoven web of fibers); polymeric materials such as apertured formed thermoplastic films, apertured plastic films, and hydroformed thermoplastic films; porous foams; reticulated foams; reticulated thermoplastic films; and thermoplastic scrims. Suitable woven and nonwoven materials can be comprised of natural fibers (e.g., wood or cotton fibers), synthetic fibers (e.g., polymeric fibers such as polyester, polypropylene, or polyethylene fibers) or from a combination of natural and synthetic fibers. When the topsheet comprises a nonwoven web, the web may be manufactured by a wide number of known techniques. For example, the web may be spunbonded, carded, wet-laid, melt-blown, hydroentangled, combinations of the above, or the like.

The backsheet is impervious to liquids (e.g., menses and/or urine) and is preferably manufactured from a thin plastic film, although other flexible liquid impervious materials may also be used. As used herein, the term "flexible" refers to materials which are compliant and will readily conform to the general shape and contours of the human body. The backsheet prevents the exudates absorbed and contained in the absorbent core from wetting articles which contact the absorbent article such as bedsheets, pants, pajamas and undergarments. The backsheet can also be vapor permeable ("breathable"), while remaining fluid impermeable. The backsheet may comprise a woven or nonwoven material, polymeric films such as thermoplastic films of polyethylene or polypropylene, or composite materials such as a film-coated nonwoven material.

The backsheet can comprise panty fastening means applied on its surface, particularly the surface facing outside the absorbent article in order to allow the article to stay in place when worn between the user's crotch and panties. Such panty fastening means can be for example a layer of adhesive or mechanical means such as Velcro® or combination thereof. When an adhesive is present, typically a release paper is also present in order to protect the adhesive before use.

The backsheet and the topsheet can positioned adjacent the garment surface and the body surface, respectively, of the absorbent core. The absorbent core can be joined with the topsheet, the backsheet, or both in any manner as is known by attachment means such as those well known in the art. Embodiments of the present invention are envisioned wherein portions of the entire absorbent core are unattached to either the topsheet, the backsheet, or both.

The absorbent core can be formed from any of the materials well known to those of ordinary skill in the art. Examples of such materials include multiple plies of creped cellulose wadding, fluffed cellulose fibers, wood pulp fibers also known as airfelt, textile fibers, a blend of fibers, a mass or batt of fibers, airlaid webs of fibers, a web of polymeric fibers, and a blend of polymeric fibers. Other suitable absorbent core materials include absorbent foams such as polyurethane foams or high internal phase emulsion ("HIPE") foams. Suitable HIPE foams are disclosed in US 5,550,167, US 5,387,207, US 5,352,711, and US 5,331,015.

For some absorbent articles, the absorbent core can be relatively thin, less than about 5 mm in thickness, or less than about 3 mm, or less than about 1 mm in thickness. Thickness can be determined by measuring the thickness at the midpoint along the longitudinal centerline of the pad by any means known in the art for doing while under a uniform pressure of 1.72 kPa.

The absorbent core can comprise superabsorbent materials such as absorbent gelling materials (AGM), including AGM fibers, as is known in the art. The absorbent core can therefore constitute a layer comprising superabsorbent material.

The absorbent article can comprise other additional components, for example between the topsheet and absorbent core, such as a secondary topsheet or acquisition layer. The secondary topsheet or acquisition layer can comprise a tissue layer or a nonwoven, such as carded resin-bonded nonwovens, embossed carded resin-bonded nonwovens, high-loft carded resin-bonded nonwovens, carded through-air-bonded nonwovens, carded thermo-bonded nonwovens, spunbonded nonwovens, and the like. A variety of fibers can be used in the secondary topsheet or acquisition layer, including natural fibers, e.g. wood pulp, cotton, wool, and the like, as well as biodegradeable fibers, such as polylactic acid fibers, and synthetic fibers such as polyolefins (e.g., polyethylene and polypropylene), polyesters, polyamides, synthetic cellulosics (e.g., RAYON®, Lyocell), cellulose acetate, bicomponent fibers, and blends thereof. The basis weight of the secondary topsheet or acquisition layer can vary depending upon the desired application.

The absorbent article can comprise further components such as side cuffs, typically found in diapers, or side wings or side flaps, typically found in sanitary napkins.

Absorbent catamenial tampons are absorbent articles for internal use in the vagina which are typically made by a pledget comprising absorbent fibers compressed to a cylindrical shape. Tampons can be "digital tampons" when they have a self sustaining shape and can be inserted with a finger or "applicator tampons" i.e. tampons which are introduced using an applicator. Tampons can also comprise an extraction cord so to facilitate extraction from the vagina.

The absorbent articles herein are preferably disposable after a single use.

Absorbent articles herein are often commercialized in packages containing a plurality of units, often the package is a plastic film or a carton box. Single units contained within the commercial package can be individually packaged or not.

The cyclodextrin complex of the present invention can be disposed in various locations in the absorbent article. The cyclodextrin complex can be disposed on the garment-facing side or the body-facing side of the topsheet or absorbent core, or the body-facing side of the backsheet. Preferably, the cyclodextrin complex is disposed on the absorbent core, and preferably on the body-facing side of the absorbent core. The malodor control composition can also be disposed on other components, when present in the absorbent article, such as the garment-facing side or body-facing side of a secondary topsheet or acquisition layer.

In certain embodiments the cyclodextrin complex of the present invention is disposed in the absorbent article in a layer that is closer to the body-facing surface of the absorbent article than the absorbent core or a layer comprising superabsorbent material (e.g. absorbent gelling material ("AGM")). In order for the cyclodextrin complex to effectively release the components of the cyclodextrin complex, the complex needs to come in contact with moisture. A problem exists when incorporating a cyclodextrin complex in an absorbent article, because other components, such as the absorbent core and/or superabsorbent material, of the absorbent article have a strong affinity for bodily fluids, including the moisture contained therein. When an absorbent article is insulted with bodily fluid, such as menses or urine, the cyclodextrin complex is thus in competition with the absorbent core and/or superabsorbent material for the moisture contained in the bodily fluid. The absorbent core and/or superabsorbent material has a strong affinity for the moisture and once the absorbent core and/or superabsorbent material contacts the bodily fluid, the absorbent core and/or superabsorbent material effectively "lock-up" the moisture of the bodily fluid, thereby reducing the amount of moisture available to contact the cyclodextrin complex and release the components of the cyclodextrin complex to provide odor control benefits. In these cases disposing the cyclodextrin complex in the absorbent article in a layer that is closer to the body-facing surface of the absorbent article than the absorbent core and/or a layer comprising superabsorbent material enables the cyclodextrin complex to come in contact with the bodily fluid preferentially before the bodily fluid comes into contact with the absorbent core and/or superabsorbent material. This results in more effective release of the components of the cyclodextrin complex and providing improved odor control benefits.

### CYCLODEXTRIN COMPLEX

As used herein, the term "cyclodextrin" includes any of the known cyclodextrins such as substituted and unsubstituted cyclodextrins containing from about six to about twelve glucose units, for example alpha-cyclodextrin, beta-cyclodextrin, gamma-cyclodextrin and/or their derivatives and/or mixtures thereof. For example, the cyclodextrin complex of the present invention can comprise cyclodextrin selected from the group consisting of beta-cyclodextrin, alpha-cyclodextrin, hydroxypropyl alpha-cyclodextrin, hydroxypropyl beta-cyclodextrin, methylated-alpha-cyclodextrin, methylated-beta-cyclodextrin, and mixtures thereof.

For "cyclodextrin complex" it is intended an "inclusion complex" within the meaning of IUPAC Compendium of Chemical Terminology 2nd Edition (1997) wherein the cyclodextrin is the host and the complexed component is the "guest".

The components complexed with the cyclodextrin can be selected from a number of different components.

The cyclodextrin complex of the present invention comprises cyclodextrin and at least two components complexed with said cyclodextrin, wherein said at least two components are:
a) a menthol family compounds selected from menthol, menthyl acetate, menthyl lactate, menthyl propionate, menthyl butyrrate, menthone, mint terpenes, laevo-carvone, Cis-3-Hexenol & Cis-3-Hexenyl acetate and mixture thereof,
b) an ionone selected from:
   1-(2,6,6-trimethyl-1-cyclohexenyl)pent-1-en-3-one;
   4-(2,6,6-trimethyl-1-cyclohexen-1-yl)-3-Buten-2-one;
   4-(2,6,6-trimethyl-2-cyclohexen-1-yl)-3-buten-2-one,
   5-(2,6,6-Trimethyl-2-cyclohexen-1-yl)4-penten-3-one,
   (E)-4-(2,2-dimethyl-6-methylidenecyclohexyl)but-3-en-2-one
   and mixture thereof

In some embodiments a third component is present selected from:
c) methyl-dihydrojasmonate, methyl jasmonate, eucalyptol, tetrahydro-linalool, Phenyl-Ethyl alcohol, Hexyl iso-butyrate, Linalyl acetate, Benzyl acetate or mixture thereof.

Components from class (a) are all compounds which primary function is to mask malodours inhibiting the receptors of the nose. When used, these materials may significantly reduce the capability for the nose to detect the malodours. The nose blocking is possible due to the volatile nature of the materials selected, which are released from the cyclodextrin complex of the absorbent article and are then inhaled into the nose of a consumer, generally within somewhat close range of the absorbent article, e.g. within about 0 to 10 meters of the article by normal breathing (although this should in no way be intended to limit the scope of the invention). The blocking of the nose receptors is, of course, only temporary. A secondary effect of components of class (a) is to mask malodours by modifying the vapour pressure of the malodourant compounds, thereby reducing the impression of the malodour.

Components from class (b) are all compounds which primary function is to chemically react with malodors, such as ammonia-based malodors, Ammonia is one component of malodor associated with the absorption bodily fluids, such as menses or urine. For example, ammonia is typically present in high amounts in absorbent products used for urine absorption due to degradation of urea. Ammonia and its derivatives can react with aldehydes and/or ketones to form imines (according to the so-called Schiff base reaction).

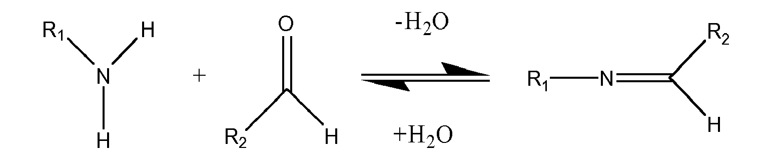

This reaction is catalyzed by enzymes and/or by a slightly acidic pH 4 to 5. The moderate acid requirement is necessary to allow protonation of the hydroxyl intermediate to allow water to leave.

Components of class (b) are also unexpectedly effective in masking malodours originating from sulphur based compounds. Malodourant sulphur based compounds are typically generated by the degradation of menstrual fluids and so their control is particularly important in menstrual absorbent articles such as sanitary napkins or pantyliners. The mechanism of action is not fully understood at the moment, but it is believed that components in class (b) have a specific effect on the nasal mucous membranes and nasal receptors in inhibiting specifically the perception of malodours originating from sulphur based compounds.

The combination of components from class (a) and (b) is particularly effective in the specific context of the present invention because compounds in both classes have a pleasant and low intensity odour, and, more importantly, are able to be complexed effectively with cyclodextrins and to be quickly released when the cyclodextrin complex gets in contact with water based liquids such as urine or menses.

Moreover the combination of the general masking effect provided by class (a) components with the specific masking effect on malodourant sulphur based compounds provided by class (b) components renders the combination particularly effective in absorbent articles which are used to absorb menses.

In some embodiment a third component (c) is present as cyclodextrin complex along with components (a) and (b).

Components from class (c) are volatile materials which are well complexed by the cyclodextrin and are release very quickly upon contact with a water based liquid. Their presence allows the absorbent article to respond more quickly to an insult of malodourant liquid by releasing a compound that have a good general masking effect against malodors, in particular, being very volatile, reduces the vapor pressure of other malodourant compounds slowing down their evaporation rate.

In all embodiments other additional components can be present as cyclodextrin complexes.

In some embodiments, components (a), (b) and, if present, (c), these together preferably represent more than 50% or more than 70% or more than 80% by weight of all components complexed with cyclodextrins (weight measured in uncomplexed form).

Suitable additional components which can be present as cyclodextrin complexes include in particular other fragrance components and reactive components. In some embodiments at least part of the additional components are selected from lists d) and e) detailed below.

Fragrance components are typically used in the field of perfumery to provide a composition with an aesthetically pleasing scent. Reactive components include components that can react with malodors, such as ammonia-based malodors (see Schiff base reaction above) or sulphur-based malodors (i.e. "malodor reactive components"), and components that mask malodors and/or react with receptors of the nose to block the perception of malodor by the nose of a consumer (i.e. "malodor masking components"). Suitable reactive components are described, for example, in US 2008/0071238 Al and WO 2007/113778 A2.

Many aldehydes and ketones capable of imine (Schiff base) reaction have an unpleasant and/or too intense odor that can be disturbing to human nose and/or they are very volatile and so not stable in the product. Therefore, selected aldehydes and/or ketones for controlling such malodors are used. Examples of suitable aldehydes and ketones for controlling malodour are those aldehydes and ketones that are able to react with amine compounds according to Schiff base reaction and have not unpleasant odor. Suitable reactive components include reactive components selected from the group d) consisting of:
d) hexyl cinnamic aldehyde, alpha-amylcinnamic aldehyde, p-anisaldehyde, benzaldehyde, cinnamic aldehyde, cuminic aldehyde, decanal, cyclamen aldehyde, p-t-butyl-alpha-methyldihydrocinnamaldehyde, 4-hydroxy-3-methoxycinnamaldehyde, vanillin isobutyrate, 2-phenyl-3-(2-furyl)prop-2-enal, ethyl vanillin acetate, vanillin acetate, heptanal, lauryl aldehyde, nonanal, octanal, phenylacetaldehyde, phenyl propyl aldehyde, vanillin, salycil aldehyde, cytral, 2,4-dihydroxy-3-methylbenzaldehyde, 2-hydroxy-4-methylbenzaldehyde, 5-methyl salicylic aldehydes, 4-nitrobenzaldehyde, o-nitrobenzaldehyde, 5-ethyl-2-thiophenecarbaldehyde, 5-methyl-2-thiophenecarboxaldehyde, 2-thiophenecarbaldehyde, asaronaldehyde, 5-(hydroxymethyl)-2-furaldehyde, 2-benzofurancarboxaldehyde, 2,3,4-trimethoxybenzaldehyde, protocatechualdehyde, heliotropine, 4-ethoxy-3-methoxy benzaldehyde, 3,4,5-trimethoxybenzaldehyde, 3-hydroxybenzaldehyde, o-methoxycinnamaldehyde, 3,5-dimethoxy-4-hydroxycinnamaldehyde, 2,8-dithianon-4-3n-4-carboxaldehyde, sorbinaldehyde, 2,4-heptadienal, 2,4-decadienal, 2,4-nonadienal, 2,4-nonadienal, (E,E)- ,2,4-octadien-1-al, 2,4-octadienal, 2,4-dodecadienal, 2,4-undecadienal, 2,4-tridecadien-1-al, 2-trans-4-cis-7-cis-tridecatrienal, piperonylidene propionaldehyde, 2-methyl-3-(2-furyl)acrolein, 2,4-pentadienal, 2-furfurylidene butyraldehyde, 3-(2-furyl)acrolein, pyruvaldehyde, ethanedial, and mixtures thereof.

Other suitable malodor masking and fragrance components include those in the following list e):
e) camphor, p-menthane, limonene, cresol, linalool, myrcenol, tetra hydromyrcenol, di-hydromyrcenol, myrcene, cytronellol, cytronellyil derivatives, geraniol, geranyl derivatives, mugetanol, eugenol, jasmal, terpineol, pinanol, cedrene, damascone, beta pinene, cineole and its derivatives, nonadienol, ethylhexanal, octanol acetate, methyl furfural, terpinene, thujene, amylacetate, camphene, citronellal, dihydrocumarin, dy hydromyrcenyl acetate, geraniol, geranial, isoamylacetate, ethyl, and/or triethyl acetate, para-cresol, para-cymene, methyl abietate, , hexyl-2-methyl butyrate, hexyl-2-methyl butyrate , , and mixtures thereof.

The materials also include their isomeric forms, diastereomers and enantiomers. Advantageously, in general, the above materials have only a very slight inherent odour but show a high degree of malodour masking and/or nose receptor blocking.

It may be that, for certain components, the same component can be considered both a malodor reactive component, a malodor masking component, and/or a fragrance component.

In some embodiments the absorbent articles of the present invention, in addition to the components from lists a), b), c), d) and e) as cyclodextrin complexes may also include components from the same lists or other fragrance components in an uncomplexed form.

In the present invention it is however preferred that the absorbent article exhibits no noticeable scent (or very little scent) before use. As a result, it is preferred that no other fragrant compounds are present and that the cyclodextrin complex has a low amount of free components that are not complexed with the cyclodextrin. In accordance with at least some of the preferred embodiments, the percent of components that are complexed with cyclodextrin is greater than about 75%, greater than about 90%, or greater than about 95%. It should be understood that these levels of component complexation are directly associated with the complex formation process itself; i.e. the percentages do not represent a formulation design of adding a first percentage of components via a cyclodextrin complex and adding a second percentage of neat components.

Cyclodextrin complexes can be formed by various methods which are well known in the art. For example, US 5,543,157 from The Procter & Gamble Company describes methods of forming cyclodextrin complexes.

As one example of a method of forming a cyclodextrin complex, a solvent (e.g., water or an organic solvent suitable for the organic compound to be complexed), unloaded cyclodextrin particles, and the organic compound which need to be complexed can be placed into a container and then mixed for a period of time to permit loading of organic molecules into "cavities" of cyclodextrin molecules. The mixture may or may not be processed further; e.g., processed through a colloid mill and/or homogenizer. The solvent is then substantially removed from the resulting mixture or slurry to yield cyclodextrin complex particles, e.g. via spray drying. Different manufacturing techniques may however impart different particle/complex characterizations, which may or may not be desirable in the absorbent articles, depending on the specific usage and conditions. In some embodiments the particles of cyclodextrin inclusion complexes have a low level of moisture prior to their inclusion into the polysiloxane carrier, typically of less than about 20% by weight of the particles, or of less than about 10% by weight of the particles, or of less than about 6% by weight of the particles. Spray drying a slurry of inclusion complexes of cyclodextrin and organic compounds is one manufacturing technique capable of producing the cyclodextrin particles and cyclodextrin complexes having the above-noted, moisture levels. Cyclodextrin complexes can also be obtained using known techniques and an extrusion process (kneading) however the resulting material will in general contain a higher humidity and a lower complexation efficiency. US 2008/0213191 Al from The Procter & Gamble Company provides a detailed overview of preferred techniques for preparing cyclodextrin complexes.

The cyclodextrin complex can be applied in a variety of ways, and in a variety of patterns, to the absorbent article. For example, when the cyclodextrin complex is dispersed in a carrier, the dispersion containing the cyclodextrin complex can be applied using conventional glue application equipment such as a slot applicator, which can be used for striped patterns, or air assisted applicators for patterned applications (like spray, spiral, serpentine, fibrils, omega®, signature® and the like) because this allow to position the odour control material in a way that it does not impact fluid acquisition (i.e. in a fem care article the material is not applied in correspondence with the vaginal opening) and the pattern, having a large void space, allows fluid penetration also on the sides. Also patterned applications are helpful because it allows a precise application so that it is easier to avoid contact with the glue which connects the various layers of the absorbent article.

The cyclodextrin complex can applied in powder form or can be incorporated into a carrier and applied as a lotion. The cyclodextrin complex can be dispersed in a carrier to form a dispersion, and the dispersion applied to the absorbent article. The carrier can be selected from the group consisting of polysiloxane oil, mineral oil, petrolatum, polyethylene glycol, glyercin, and the like, and mixtures thereof. The carrier is preferably polysiloxane oil, such as a silicone glycol copolymer (commercially available from Dow Coming as Dow Coming 190 Fluid).

The cyclodextrin complex is typically disposed in the absorbent article in an amount of from about 10 to about 5000 milligrams per absorbent article, from about 20 to about 1000 milligrams per absorbent article, from about 30 to about 500 milligrams per absorbent article, or from about 70 to about 300 milligrams per absorbent article.

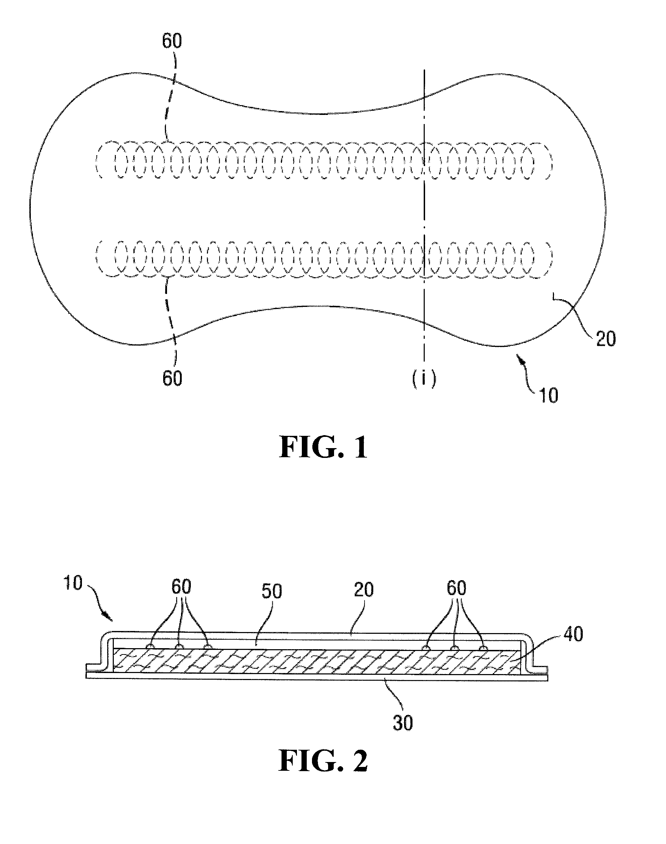

FIG. 1 shows an absorbent article, such as a sanitary napkin, according to the present invention. FIG. 2 is a cross-sectional view of the same absorbent article along the line indicated by (i) in FIG. 1. The absorbent article (10) comprises a topsheet (20), a backsheet (30), an absorbent core (40), a secondary topsheet (50) and two spirals of cyclodextrin complex (60) according to the present invention applied to the body-facing surface of the absorbent core (40). The cyclodextrin complex (60) is therefore disposed in a layer of the absorbent article (10) that is closer to the body-facing surface of the absorbent article (10) than the absorbent core (40).

The present invention further encompasses a method of reducing malodor associated with bodily fluid such as urine, menses, and/or feces, comprising the step of contacting the bodily fluid with an absorbent article of the present invention. Preferably, the method reduces the malodor associated with menses.

### EXAMPLE 1

This is an example of an absorbent article of the present invention wherein the cyclodextrin complex is disposed on the garment-facing side of the secondary topsheet of the absorbent article.

The cyclodextrin complex is prepared as follows. The following components are added in order in a mildly agitated vessel, to create movement at the top of fluid, but without creating air bubbles: 55 grams of distilled water, 41 grams of beta cyclodextrin (contains nominally 12% moisture), and 4 grams of the Component Mixutre of Table 1 below.

| TABLE 1: COMPONENT MIXTURE | |
|---|---|
| INGREDIENT | AMOUNT (wt%) |
| Menthyl Acetate | 20.000 |
| Xandralia 992420 ¹ | 20.000 |
| Methyl Dihydro Jasmonate | 30.000 |
| Benzyl Acetate | 7.000 |
| Tetra Hydro Linalool | 9.000 |
| Laevo Carvone | 0.200 |
| Hexyl-2-methyl Butyrate | 2.000 |
| Eucalyptus | 0.300 |
| Linalyl Acetate | 3.500 |
| Phenyl Ethyl Alcohol | 8.000 |

| | |
|---|---|
| ¹ available from Firmenich | |

The resulting slurry is agitated for 30 minutes and then passed through a colloid mill (Gaulin mill). The rheology of the solution changes to a viscous slurry as the complexation occurs. The slurry is then dried via nozzle spray drying at an inlet temperature of approximately 195°C and an outlet temperature of about 98°C. The resulting cyclodextrin complex is a powder having a moisture content of about 5%, by weight of the cyclodextrin complex, and a content of components complexed with cyclodextrin of about 8% to about 9%, by weight of the cyclodextrin complex. The cyclodextrin complex has less than about 2% of components that are uncomplexed with the cyclodextrin.

A LINES PETALO BLU CON ALI sanitary napkin, commercially available from Fater SpA, Italy, is obtained. The release paper wrapper of the sanitary napkin is removed and the sanitary napkin is unfolded into a flat, unfolded configuration. The sanitary napkin is then cut along one longitudinal side of the article (leaving the other longitudinal side intact). The topsheet is separated from the secondary topsheet ("STS"). On the garment-facing side of the STS, 50 milligrams of the cyclodextrin complex is applied in the center of the STS in an area of 3 cm x 8 cm (a spatula is used to apply the cyclodextrin complex uniformly). The sanitary napkin is reassembled in its original order and orientation, and a new thermal seal is provided along the cut longitudinal side.

### EXAMPLE 2

This is an example of an absorbent article of the present invention wherein the cyclodextrin complex is formulated with a carrier and disposed on the garment-facing side of the secondary topsheet of the absorbent article.

A cyclodextrin complex is prepared as described in Example 1. 40 grams of the cyclodextrin complex are added slowly to 60 grams of a silicon glycol copolymer (Dow Coming 190 Fluid) in a mixer while stirring, obtaining a homogeneous dispersion which is kept under stirring.

A sanitary napkin, ALWAYS Ultra Regular available from The Procter & Gamble Company, is cut along a longitudinal side (leaving the other longitudinal side intact). The topsheet is separated from the secondary topsheet ("STS"). On the garment-facing side of the STS, 170 milligrams of the dispersion containing Dow Coming 190 Fluid and the cyclodextrin complex is applied in two thin spirals similar to those shown in Fig. 1. The sanitary napkin is reassembled in its original order and orientation, and a new thermal seal is provided along the cut longitudinal side.

## Claims

1. An absorbent article comprising:
a cyclodextrin complex comprising cyclodextrin and at least two components complexed with said cyclodextrin, wherein said at least two components are:
a) a menthol family compound selected from menthol, menthyl acetate, menthyl acetate, menthyl lactate, menthyl propionate, menthyl butyrrate, menthone, mint terpenes, laevo-carvone, Cis-3-Hexenol & Cis-3-Hexenyl acetate and mixture thereof,
b) an ionone selected from:
1-(2,6,6-trimethyl-1-cyclohexenyl)pent-1-en-3-one;
4-(2,6,6-trimethyl-1-cyclohexen-1-yl)-3-Buten-2-one;
4-(2,6,6-trimethyl-2-cyclohexen-1-yl)-3-buten-2-one,
5-(2,6,6-Trimethyl-2-cyclohexen-1-yl)4-penten-3-one,
(E)-4-(2,2-dimethyl-6-methylidenecyclohexyl)but-3-en-2-one and mixture thereof

2. The absorbent article of Claim 1, wherein said cyclodextrin complex comprises at least three components complexed with said cyclodextrin, wherein the third component is a boosting compound selected from:
c) methyl-dihydrojasmonate, methyl jasmonate, eucalyptol, tetrahydro-linalool, Phenyl-Ethyl alcohol, Hexyl iso-butyrate, Linalyl acetate, Benzyl acetate or mixture thereof.

3. The absorbent article of claim 1 or 2 wherein said cyclodextrin complex comprises one or more other components complexed with said cyclodextrin which are selected from the following lists:
d) reactive components selected from the group consisting of hexyl cinnamic aldehyde, alpha-amylcinnamic aldehyde, p-anisaldehyde, benzaldehyde, cinnamic aldehyde, cuminic aldehyde, decanal, cyclamen aldehyde, p-t-butyl-alpha-methyldihydrocinnamaldehyde, 4-hydroxy-3-methoxycinnamaldehyde, vanillin isobutyrate, 2-phenyl-3-(2-furyl)prop-2-enal, ethyl vanillin acetate, vanillin acetate, heptanal, lauryl aldehyde, nonanal, octanal, phenylacetaldehyde, phenyl propyl aldehyde, vanillin, salycil aldehyde, cytral, 2,4-dihydroxy-3-methylbenzaldehyde, 2-hydroxy-4-methylbenzaldehyde, 5-methyl salicylic aldehydes, 4-nitrobenzaldehyde, o-nitrobenzaldehyde, 5-ethyl-2-thiophenecarbaldehyde, 5-methyl-2-thiophenecarboxaldehyde, 2-thiophenecarbaldehyde, asaronaldehyde, 5-(hydroxymethyl)-2-furaldehyde, 2-benzofurancarboxaldehyde, 2,3,4-trimethoxybenzaldehyde, protocatechualdehyde, heliotropine, 4-ethoxy-3-methoxy benzaldehyde, 3,4,5-trimethoxybenzaldehyde, 3-hydroxybenzaldehyde, o-methoxycinnamaldehyde, 3,5-dimethoxy-4-hydroxycinnamaldehyde, 2,8-dithianon-4-3n-4-carboxaldehyde, sorbinaldehyde, 2,4-heptadienal, 2,4-decadienal, 2,4-nonadienal, 2,4-nonadienal, (E,E)- ,2,4-octadien-1-al, 2,4-octadienal, 2,4-dodecadienal, 2,4-undecadienal, 2,4-tridecadien-1-al, 2-trans-4-cis-7-cis-tridecatrienal, piperonylidene propionaldehyde, 2-methyl-3-(2-furyl)acrolein, 2,4-pentadienal, 2-furfurylidene butyraldehyde, 3-(2-furyl)acrolein, pyruvaldehyde, ethanedial, and mixtures thereof,
e) malodor masking components selected from the group consisting of camphor, p-menthane, limonene, cresol, linalool, myrcenol, tetra hydromyrcenol, di-hydromyrcenol, myrcene, cytronellol, cytronellyil derivatives, geraniol, geranyl derivatives, mugetanol, eugenol, jasmal, terpineol, pinanol, cedrene, damascone, beta pinene, cineole and its derivatives, nonadienol, ethylhexanal, octanol acetate, methyl furfural, terpinene, thujene, amylacetate, camphene, citronellal, dihydrocumarin, dy hydromyrcenyl acetate, geraniol, geranial, isoamylacetate, ethyl, and/or triethyl acetate, para-cresol, para-cymene, methyl abietate, , hexyl-2-methyl butyrate, hexyl-2-methyl butyrate , and mixtures thereof.

4. The absorbent article of any preceding claim wherein, considering all the components complexed by the cyclodextrin, the components a) b) and, if present, c) represent together more than 50%, preferably more than 70%, more preferably more than 80% by weight of all complexed components.

5. The absorbent article of any one of the preceding claims, wherein said cyclodextrin is selected from the group consisting of alpha-cyclodextrin and beta-cyclodextrin.

6. The absorbent article of any one of the preceding claims, wherein said cyclodextrin complex is dispersed in a polysiloxane oil to form a dispersion, wherein said dispersion is applied to said absorbent article.

7. The absorbent article of any one of the preceding claims, wherein said absorbent article comprises:
a topsheet;
a backsheet;
an absorbent core disposed between said topsheet and said backsheet.

8. The absorbent article of claim 7 wherein the backsheet comprises panty fastening means applied onto its surface.

9. The absorbent article of claims 7-8 wherein said absorbent article is a sanitary napkin or a pantyliner.

10. The absorbent article of claims 7-9, wherein said absorbent article further comprises a layer of material disposed between said topsheet and said absorbent core and wherein said cyclodextrin complex is disposed on said layer of material disposed between said topsheet and said absorbent core.

11. The absorbent article of Claim 10, wherein said layer of material disposed between said topsheet and said absorbent core is a secondary topsheet.

12. The absorbent article of Claim 11, wherein said secondary topsheet has a body-facing surface and a garment-facing surface, wherein said cyclodextrin complex is disposed on said garment-facing surface of said secondary topsheet.

13. The absorbent article of claims 1-6, wherein said absorbent article is a catamenial tampon comprising an extraction cord.

14. A package comprising a plurality of absorbent articles according to any preceding claim.

15. A method of reducing malodor associated with menses comprising the step of contacting said menses with an absorbent article of any one claims 1-13.
